# FASCICULE DE BREVET EUROPEEN

(11) **EP 2 563 267 B1**
(45) Date de publication et mention de la délivrance du brevet: **23.03.2016**
(21) Numéro de dépôt: 11723505.1
(22) Date de dépôt: 22.04.2011
(51) Int. Cl.: A61C 8/00, A61B 50/20, A61B 50/30, A61B 17/86

(54) **EMBALLAGE POUR OBJET ET ENSEMBLE COMPRENANT UN TEL EMBALLAGE**
VERPACKUNG FÜR EIN OBJEKT UND SET MIT SOLCH EINER VERPACKUNG
PACKAGING FOR AN OBJECT AND SET COMPRISING SUCH PACKAGING

(30) Priorité: 27.04.2010 FR 1053191
(43) Date de publication de la demande: 06.03.2013
(73) Titulaire: Selenium Medical, 17000 La Rochelle (FR)
(72) Inventeur: RICHART, Olivier, 17140 Lagord (FR)
(74) Mandataire: Godineau, Valérie
(86) Numéro de dépôt international: PCT/FR2011/050943
(87) Numéro de publication internationale: WO 2011/135246

(56) Documents cités:
- WO-A1-00/02496
- DE-A1- 10 146 905
- US-A- 5 062 800

## Description

La présente invention concerne de manière générale les emballages pour objets, en particulier pour pièces médicales de préférence stérilisées.

On connaît de l'état de la technique des emballages se présentant sous la forme de sachets ou de coques plastiques blister dans lesquels un objet est contenu. Dans le cas de pièces médicales devant être déconditionnées dans des conditions proches de l'asepsie, de tels emballages posent des problèmes de contamination lors du passage de l'emballage d'une personne à une autre et de l'ouverture dudit emballage. En effet, depuis son départ d'une zone de travail dite sale, c'est-à-dire sans condition d'asepsie particulière, jusqu'à son arrivée dans une zone de travail dite propre, c'est-à-dire une zone de travail dans laquelle des conditions d'asepsie données sont respectées, par exemple la zone du bloc opératoire, l'emballage qui arrive en zone propre est contaminé au niveau de son enveloppe extérieure, ce qui présente un risque de contamination de l'objet médicale en sortant celle-ci de l'emballage.

Pour limiter ce risque de contamination, il est connu d'ouvrir le sachet de manière à faire tomber l'objet sur un plan de travail pour ne pas avoir à toucher ledit objet. Seul le chirurgien ou l'assistant stérile prend l'objet. Cependant, l'objet risque non seulement de se détériorer sous le choc avec le plan de travail, dans le cas d'un objet solide, ou encore de tomber par terre en roulant, mais également d'être contaminé par des corps étrangers présents sur le plan de travail.

Le document US5062800 décrit un emballage médical pour implant dentaire qui comprend un élément support muni de moyens de fixation de l'implant et un bouchon couplable à l'élément support de telle sorte que l'élément support s'étend en saillie dudit bouchon. Ledit emballage comprend également une coiffe qui recouvre le bouchon. Cependant, la coiffe recouvre entièrement la longueur du bouchon de sorte que l'opérateur n'a pas d'autre solution que de vider le contenu de la coiffe en le faisant tomber sur un plan de travail. Comme rappelé ci-dessus, le fait de faire tomber l'implant peut le détériorer et augmente le risque de contamination.

La présente invention a pour but de proposer un emballage pour le conditionnement et le déconditionnement d'objet qui permet de limiter les risques de contamination de l'objet tout en permettant un déconditionnement de l'objet sans avoir à le lâcher ou le toucher directement.

A cet effet, l'invention concerne un emballage pour objet, tel qu'une pièce médicale de préférence stérilisée, caractérisé en ce que ledit emballage comprend :
- un élément support muni de moyens pour le couplage dudit objet audit élément support,
- un premier élément de protection creux, appelé bouchon, couplable à l'élément support dudit objet, de préférence par emboitement à recouvrement partiel, de telle sorte que l'élément support présente une partie, dite partie de saisie, qui s'étend en saillie dudit bouchon,
   ledit élément support délimitant à lui seul ou en coopération avec le bouchon une première chambre renfermant ledit objet,
- un deuxième élément de protection creux, appelé coiffe,
caractérisé en ce que ladite coiffe est couplable au bouchon, de préférence par emboitement à recouvrement partiel, de manière à délimiter en coopération avec ledit bouchon une deuxième chambre à l'intérieur de laquelle s'étend ladite partie de saisie de l'élément support,
et en ce que, à l'état couplé de la coiffe au bouchon, une partie, dite partie de saisie, du bouchon s'étend en saillie de la coiffe.

Grâce à la partie de saisie de chaque élément de protection et de l'élément support, qui s'étend en saillie de l'élément de protection avec lequel il est couplé, les chambres définies par emboitement à recouvrement partiel desdits éléments peuvent être ouvertes jusqu'à accéder à l'objet sans jamais lâcher ou toucher ledit objet et avec une risque réduit de contamination de l'objet.

En effet lors du passage de l'emballage d'un premier à un deuxième opérateur, la coiffe de protection peut être retirée de manière à ouvrir la première chambre pour découvrir l'extrémité de saisie de l'élément support de l'objet et ne conserver pour le deuxième opérateur que le sous-ensemble formé du bouchon et de l'élément support.

Le deuxième opérateur tenant ledit sous-ensemble par l'extrémité du bouchon opposée à l'élément support, l'extrémité de l'élément support découverte par l'ouverture de la première chambre est propre et peut être tendue au dernier opérateur, par exemple le chirurgien. Le dernier opérateur est celui qui va saisir l'objet à l'aide d'un instrument pour l'utiliser.

Bien entendu, le premier et le deuxième opérateur peuvent être un seul et même opérateur qui retire la coiffe du bouchon en saisissant d'une main la coiffe et de l'autre le bouchon pour découvrir l'élément support.

Le bouchon et l'élément support peuvent alors être séparés de manière à ouvrir la chambre contenant l'objet qui reste couplé à l'élément support tenu par le dernier opérateur. Le dernier opérateur qui ne conserve ainsi que l'élément support auquel est couplé l'objet peut alors de sa main propre qui tient l'extrémité de saisie de l'élément support, désactiver les moyens de couplage et saisir l'objet, par exemple à l'aide d'un instrument, par son autre main propre.

Une telle conception de l'emballage selon l'invention permet de réaliser un emballage avec un nombre de pièces réduit, principalement trois, de telle sorte que, lors du passage de l'emballage depuis une zone sale jusqu'à une zone propre par l'intermédiaire de différentes personnes, ni l'élément support ni l'objet ne sont touchés par une personne en zone sale et l'objet couplé à l'élément support n'est jamais lâché. Le risque de contamination est ainsi très fortement réduit.

Selon une caractéristique avantageuse de l'invention, le bouchon et la coiffe sont formés chacun par un corps creux allongé ouvert à une extrémité et fermé à l'autre extrémité.

Le caractère allongé des éléments de protection de l'objet formés par la coiffe et le bouchon permet de bénéficier d'une distance de sécurité lors de l'ouverture des chambres au cours du passage des différentes parties de l'emballage d'une personne à une autre. En effet, deux personnes peuvent saisir l'enveloppe extérieure de l'emballage formée par le bouchon et la coiffe ou le sous-ensemble de l'emballage formé par le bouchon et l'élément support, en les saisissant chacun à une extrémité opposée, ce qui réduit le risque de contamination.

Selon une caractéristique avantageuse de l'invention, les moyens pour le couplage de l'objet à l'élément support comprennent un corps déformable, appelé valve, présentant un orifice, de préférence de forme ovale, apte à passer, par déformation dudit corps, d'une configuration dite de rétention dans laquelle ledit orifice de la valve présente une forme et/ou des dimensions permettant d'enserrer l'objet, à une configuration dite de libération dans laquelle ledit orifice présente une forme et/ou des dimensions permettant de libérer ledit objet par rapport audit orifice de la valve.

Selon un autre mode de réalisation particulier des moyens de couplage, du type pour lequel ledit objet présente un corps allongé, lesdits moyens pour le couplage de l'objet à l'élément support comprennent un corps allongé déformable, appelé mandrin, le long et à l'intérieur duquel est ménagé un orifice d'insertion de l'objet. Lesdits moyens comprennent également un soufflet, entourant le mandrin, apte à passer d'une position déployée des plis de soufflet dans laquelle ledit soufflet appuie sur le mandrin de manière à resserrer l'orifice d'insertion autour de l'objet, à une position comprimée desdits plis de soufflet dans laquelle le mandrin est libre de contrainte par rapport au soufflet de manière à permettre le déplacement de l'objet par rapport audit orifice d'insertion du mandrin.

Préférentiellement, ledit élément support comprend au moins deux ailettes écartées l'une de l'autre et qui s'étendent de part et d'autre et le long de l'ensemble formé du mandrin et du soufflet.

Selon un autre mode de réalisation particulier des moyens de couplage, les moyens pour le couplage de l'objet à l'élément support présentent une configuration d'emprisonnement de l'objet dans une orientation donnée dudit objet et une configuration de libération par pivotement dudit objet.

Selon un autre mode de réalisation particulier des moyens de couplage, l'élément support et les moyens pour le couplage de l'objet à l'élément support sont configurés pour maintenir l'objet suspendu à travers lesdits moyens de couplage, de manière à permettre de visualiser la longueur dudit objet à l'état couplé dudit objet à l'élément support.

Selon une caractéristique avantageuse de l'invention, lesdits éléments de support et de protection de l'emballage s'emboitent à recouvrement partiel entre eux par friction et/ou par vissage.

Selon un mode de réalisation particulier dudit emballage pour lequel ledit élément support délimite à lui seul ladite première chambre renfermant ledit objet, ledit élément support est formé d'au moins deux pièces couplables/désaccouplables l'une par rapport à l'autre, de préférence par emboitement à recouvrement partiel, pour former ladite première chambre renfermant ledit objet.

Selon un autre mode de réalisation particulier dudit emballage pour lequel ledit élément support délimite en coopération avec le bouchon ladite première chambre renfermant ledit objet, ledit élément support délimite une cavité ouverte destinée à être refermée par ledit bouchon.

Préférentiellement, ledit bouchon, et éventuellement la coiffe, présente(nt) au moins un emplacement prédéfini de positionnement d'au moins un doigt d'une personne.

Avantageusement, l'élément support et/ou le bouchon et/ou la coiffe présente(nt) une partie, de préférence formée par une membrane, imperméable aux bactéries mais perméable aux gaz pour permettre une stérilisation gazeuse de ladite première chambre et/ou de ladite deuxième chambre.

L'invention concerne également un procédé de déballage d'un objet médical, de préférence stérilisé, contenu dans un emballage tel que décrit ci-dessus, caractérisé en ce que ledit procédé comprend les étapes suivantes :
a)- séparation de la coiffe par rapport au bouchon par une première personne de manière à découvrir l'élément support,
b)- saisie de l'élément support par une deuxième personne distincte de ladite première personne,
c)- séparation dudit élément support par rapport au bouchon tenu par la première personne.

Bien entendu, on peut prévoir que la séparation de la coiffe et du bouchon soit réalisée par deux personnes différentes en zone sale au lieu d'une seule, l'une tenant une extrémité du bouchon et l'autre une extrémité de la coiffe.

Avantageusement, ledit procédé comprend les étapes supplémentaires suivantes :
d)- éventuellement, mise en attente dudit élément support qui renferme l'objet dans une zone d'attente, en attente de l'utilisation dudit objet,
e)- ouverture de la première chambre définie par ledit élément support pour en extraire ledit objet.

L'invention concerne également un ensemble comprenant un objet, tel qu'une pièce médicale de préférence stérilisée, et un emballage tel que décrit ci-dessus contenant ledit objet couplé audit élément support.

Préférentiellement, ledit objet présentant une extrémité servant à sa préhension par exemple par un instrument, ledit objet est couplé à l'élément support de telle sorte que son extrémité de préhension est libre et orientée du côté opposé à la partie de saisie de l'élément support.

L'invention sera bien comprise à la lecture de la description suivante d'exemples de réalisation, en référence aux dessins annexés dans lesquels :

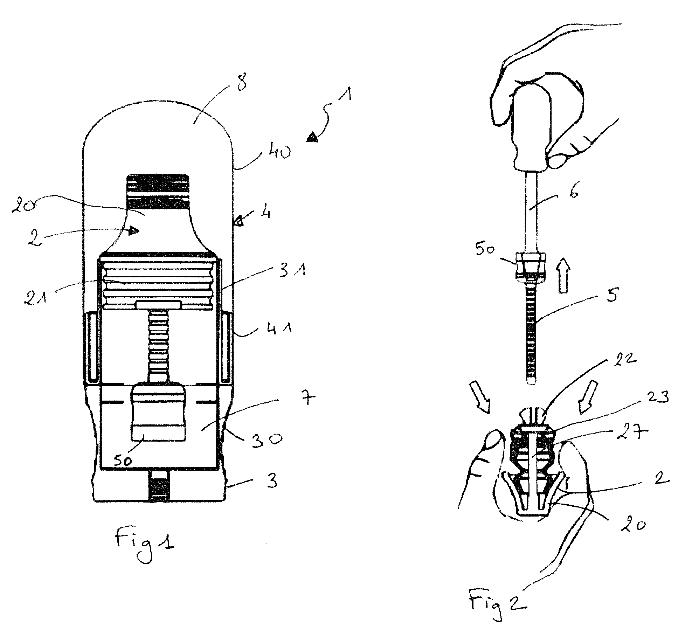

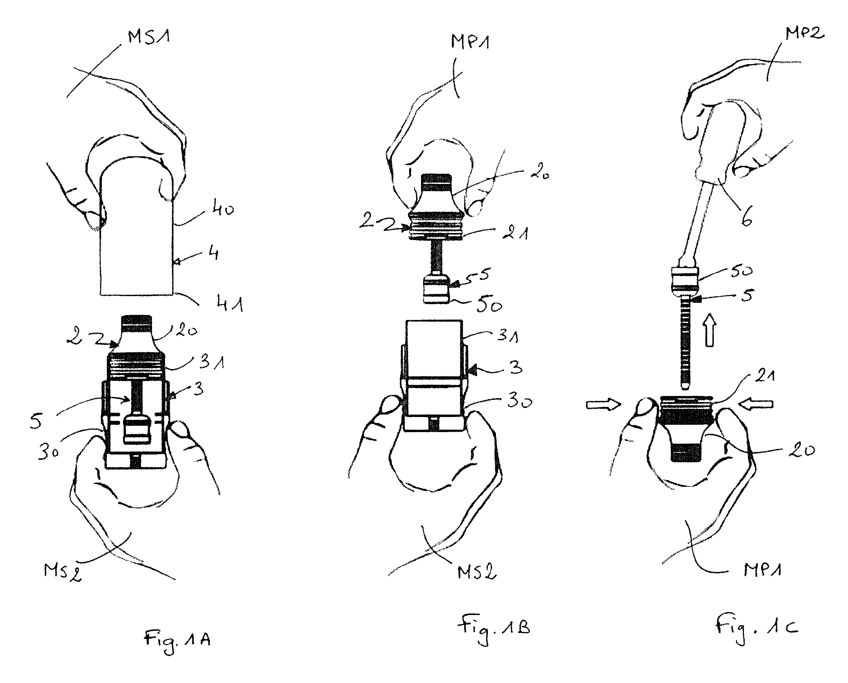

- la figure 1 est une vue d'un emballage selon l'invention conformément à un premier mode de réalisation à l'intérieur duquel est conditionnée une pièce médicale ;
- la figure 1A est une vue de l'emballage de la figure 1 au cours d'une première étape de déconditionnement pour laquelle la coiffe est retirée en restant dans la main d'un premier opérateur en zone dite sale, et le sous-ensemble restant formé du bouchon et du support de pièce est maintenu côté bouchon par la main d'un deuxième opérateur en zone sale ;
- la figure 1B est une vue du sous-ensemble de l'emballage de la figure 1A au cours d'une deuxième étape de déconditionnement pour laquelle le bouchon est retiré en restant dans la main du deuxième opérateur en zone dite sale, et le support de pièce est maintenu du côté opposé à la pièce médicale par la main d'un troisième opérateur en zone propre ;
- la figure 1C est une vue de l'élément support de l'emballage de la figure 1 B au cours d'une troisième étape de déconditionnement pour laquelle le troisième opérateur, qui tient d'une main l'élément support, vient saisir de son autre main, à l'aide d'un outil, la pièce médicale pour l'extraire de l'élément support en désactivant les moyens de couplage de la pièce médicale à l'élément support;
- la figure 2 est une vue en coupe de l'élément support selon un deuxième mode de réalisation de l'emballage pour lequel les moyens de couplage de la pièce médicale à l'élément support sont formés d'un mandrin entouré d'un soufflet ;

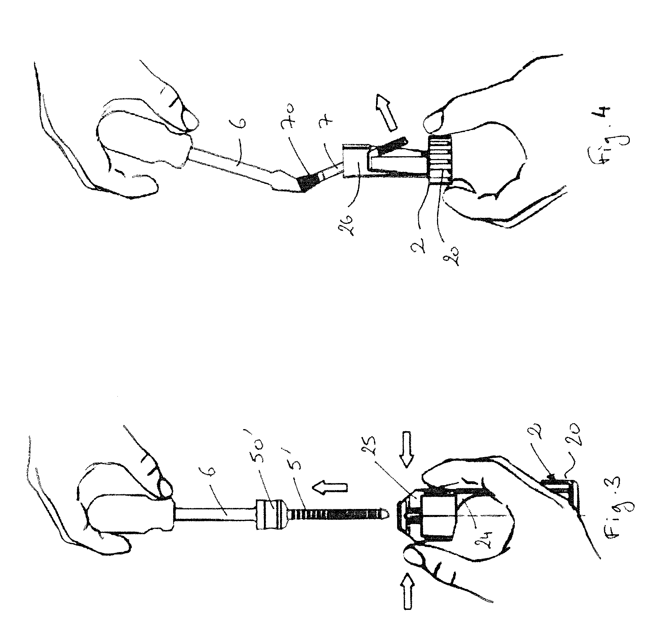

- la figure 3 est une vue en coupe de l'élément support selon un troisième mode de réalisation de l'emballage pour lequel les moyens de couplage de la pièce médicale à l'élément support sont formés par un élément déformable de type valve dont l'orifice est configuré pour maintenir par serrage la pièce médicale qui le traverse pour son couplage et pour libérer ladite pièce par déformation de ladite valve ;
- la figure 4 est une vue en coupe de l'élément support selon un quatrième mode de réalisation de l'emballage pour lequel les moyens de couplage de la pièce médicale à l'élément support sont formés par un élément présentant un logement apte à maintenir la pièce dans une orientation donnée et à permettre sa libération par rotation ou basculement, dégagement latéral de ladite pièce.

On entend par personne "sale" ou "contaminée" une personne travaillant dans des conditions non aseptisées, qui est susceptible de contaminer par ses mains les objets qu'elle touche. A l'inverse, on entend par personne "propre" une personne travaillant dans des conditions d'asepsie suffisante.

En référence aux figures et comme rappelé ci-dessus, l'invention concerne un emballage 1 pour objet, tel qu'une pièce médicale, à des fins de préservation du caractère stérile de l'objet et en vue du déballage dudit objet dans des conditions aseptiques ou proches de l'asepsie. Dans l'exemple illustré aux figures, ledit objet est une vis pour chirurgie du rachis cervical ou une vis pour chirurgie du rachis lombaire, mais l'invention s'applique également à d'autres types d'objets, en particulier à d'autres types d'implants.

Ledit emballage comprend un élément support 2 muni de moyens 21 ; 22, 23 ; 24, 25 ; 26 pour le couplage dudit objet 5 audit élément support 2 et un premier élément de protection 3 creux, appelé bouchon, couplable à l'élément support 2 dudit objet 5 par emboitement à recouvrement partiel.

A l'état couplé du bouchon 3 à l'élément support 2, ledit élément support 2 et le bouchon 3 délimitent entre eux une première chambre 7 contenant ledit objet 5. En outre, le bouchon 3 recouvre partiellement l'élément support 2 de telle sorte que la partie d'extrémité 20 libre, dite extrémité de saisie, de l'élément support 2, s'étende en saillie dudit bouchon 3 de sorte que ladite partie d'extrémité libre 20 forme une zone de saisie.

Lorsqu'il est précisé que l'élément support 2 est muni de moyens pour le couplage dudit objet 5 audit élément support 2, on entend par couplage que ledit objet n'est pas séparable dudit élément support sans action spécifique. On peut prévoir que le couplage dudit objet à l'élément support soit formé par une restriction de passage à l'intérieur de ladite première chambre de manière à empêcher ledit objet de s'échapper dudit élément support. Avantageusement, on peut prévoir que lesdits moyens de couplage comprennent également des moyens sécables permettant de libérer l'objet de l'élément support.

Ledit emballage comprend également un deuxième élément de protection 4 creux, appelé coiffe, couplable au bouchon 3 par emboitement à recouvrement partiel en venant coiffer l'extrémité 31 dudit bouchon 3 qui recouvre elle même l'élément support 2 de l'objet 5. Le bouchon 3 et la coiffe 4 sont emboités à recouvrement partiel de manière à délimiter une deuxième chambre 8 à l'intérieur de laquelle s'étend l'extrémité de saisie 20 de l'élément support 2, et de telle sorte que l'extrémité 30 du bouchon 3, opposée à l'extrémité 31 dudit bouchon 3 qui recouvre l'élément support 2, s'étende en saillie de la coiffe 4 de sorte que ladite partie d'extrémité libre 30 du bouchon 3 forme une zone de saisie. L'extrémité 40 de la coiffe 4 opposée au bouchon 3 forme également une partie de saisie de l'emballage opposée à l'extrémité 30 du bouchon.

Ainsi, chaque élément de l'emballage, à savoir l'élément support 2, le bouchon 3 et la coiffe 4, présente une extrémité de saisie 20, 30, 40 par rapport à son extrémité opposée emboitée avec un autre élément de l'emballage. Lesdits éléments s'emboitent entre eux de manière à définir entre deux éléments emboités une chambre 7, 8 de préférence étanche au moins aux bactéries. Ainsi, les éléments emboités de l'emballage définissent deux chambres 7, 8 dont une 7, définie entre le bouchon et la coiffe, à l'intérieur de laquelle est logé l'objet et à laquelle on ne peut accéder qu'une fois ouverte l'autre chambre 8 servant de protection de l'extrémité de saisie 20 de l'élément support 2.

Une telle conception de l'emballage permet de le manipuler sans risque de contamination de l'objet contenu dans la chambre de présentation et sans avoir à lâcher ledit objet.

En effet, l'emballage tenu par l'extrémité 40 de la coiffe 4 par une première personne en zone salle peut être tendu à une deuxième personne également en zone sale qui saisit l'extrémité opposée 30 de l'emballage formée par l'extrémité libre du bouchon 3. La deuxième personne tire sur le bouchon 3 de sorte que la coiffe 4 reste dans la main de la première personne, ce qui permet de découvrir une zone de saisie propre 20 de l'élément support 2 opposée à l'extrémité 30 saisie par la deuxième personne. En effet, cette zone de saisie propre 20 de l'élément support 2 était précédemment logée dans la chambre 7 délimitée entre la coiffe 4 et le bouchon 3. Puis ladite deuxième personne tend le sous-emballage restant à une troisième personne en zone propre qui saisit alors l'extrémité propre 20 de l'élément support 2 qui s'étend en saillie du bouchon 3. Comme précédemment, la troisième personne tire sur l'élément support 2 de manière à ne conserver en main que ledit élément support 2 et l'objet couplé audit élément support. Avec son autre main et à l'aide d'un instrument adapté, l'opérateur extrait l'objet de son élément support 2 comme détaillé ci-après. En variante, la première et la deuxième personne peuvent être la même personne.

Préférentiellement, le bouchon 3 et la coiffe 4 sont formés chacun par un corps creux allongé ouvert à une extrémité et fermé à l'autre extrémité, c'est-à-dire un tube fermé à une extrémité. La conception de chacun des éléments de protection sous forme d'un corps allongé à la manière d'une éprouvette permet de bénéficier d'une distance de sécurité entre l'extrémité de saisie 40 de la coiffe 4 et l'extrémité de saisie 30 du bouchon 3.

Une telle distance entre les extrémités de saisie 30, 40 de l'emballage permet de limiter le risque de contamination au niveau de l'extrémité de saisie 20 de l'élément support 2 qui s'étend dans la chambre 8, lorsque ladite chambre 8 est ouverte par séparation de la coiffe 4 et du bouchon 3. De même la distance entre l'extrémité de saisie 30 du bouchon 3 et l'extrémité de saisie 20 de l'élément support 2 permet de limiter le risque de contamination lors du passage de l'élément support 2 à un opérateur "propre", stérile, qui peut ainsi saisir l'élément support par son extrémité libre 20 éloignée de l'extrémité 30 potentiellement contaminée du bouchon.

Dans l'exemple illustré aux figures 1, 1A, 1B, 1C, les moyens 21 pour le couplage de l'objet à l'élément support comprennent un corps déformable 21, appelé valve, présentant un orifice, de préférence de forme ovale, apte à passer, par déformation latérale dudit corps par rapport à l'axe dudit orifice, d'une configuration dite de rétention dans laquelle ledit orifice de la valve 21 présente une forme et/ou des dimensions permettant d'enserrer l'objet 5, à une configuration dite de libération dans laquelle ledit orifice présente une forme et/ou des dimensions permettant de libérer ledit objet 5 par rapport audit orifice de la valve 21.

A l'état non sollicité, l'orifice de la valve 21 est naturellement en position de rétention. Ainsi, dans le cas d'un orifice de forme ovale ou en ellipse à l'état naturel, c'est-à-dire sans contrainte, la déformation dudit orifice, par appui latéral, sur les sommets opposés de l'ellipse ou de l'ovale, déforme l'orifice ovale ou en ellipse en un orifice sensiblement circulaire de diamètre supérieur au plus petit diamètre de l'ellipse ou de l'ovale, ce qui permet de libérer l'objet qui n'est plus contraint par la paroi de l'orifice. Bien entendu, l'orifice de la valve est dimensionné de telle sorte que, à l'état non déformé, son plus petit diamètre soit inférieur au diamètre de l'objet mais supérieur, à l'état déformé, audit diamètre de l'objet.

Selon un autre mode de réalisation des moyens de couplage illustré à la figure 2, qui trouve une application avantageuse pour un objet présentant un corps allongé, lesdits moyens 22, 23 pour le couplage de l'objet à l'élément support comprennent un corps allongé déformable 22, appelé mandrin, le long et à l'intérieur duquel est ménagé un orifice d'insertion de l'objet.

Lesdits moyens 22, 23 comprennent également un soufflet 23, enfilé sur le mandrin 22, apte passer d'une position déployée des plis de soufflet dans laquelle ledit soufflet appuie sur le mandrin 22 de manière à resserrer l'orifice d'insertion autour de l'objet 5, à une position comprimée desdits plis de soufflet dans laquelle le mandrin 22 est libre de contrainte par rapport au soufflet de sorte que l'orifice est desserré et l'objet est libre de déplacement par rapport à l'orifice.

L'orifice du mandrin 22 est dimensionné pour, en l'absence de sollicitation du mandrin, présenter une ouverture suffisamment grande afin de recevoir le corps allongé de l'objet sans dégradation ou abrasion du mandrin.

En configuration déployée du soufflet le long du mandrin, c'est-à-dire en configuration libre des plis de soufflet, ledit soufflet appuie par sa partie supérieure sur la partie haute du mandrin, de sorte que la paroi interne de l'orifice du mandrin se resserre sur le corps de l'objet, au niveau de l'ouverture d'insertion du corps dans le mandrin.

Pour le couplage de l'objet à l'ensemble formé du mandrin et du soufflet, on comprime le soufflet pour ne pas contraindre l'orifice du mandrin afin de permettre l'introduction du corps de l'objet dans ledit orifice, puis le soufflet est relâché de sorte qu'il appuie sur le mandrin de manière à resserrer l'orifice autour du corps dudit mandrin. La libération de l'objet s'effectue en comprimant le soufflet pour ne plus contraindre l'orifice autour de l'objet et permettre son extraction à l'aide d'un instrument.

Préférentiellement, comme illustré à la figure 2, ledit élément support 2 comprend également au moins deux ailettes 27 écartées l'une de l'autre et qui s'étendent de part et d'autre et le long de l'ensemble formé du mandrin 22 et du soufflet 23.

Lesdites ailettes prennent naissance au niveau de la partie de l'élément support munie des moyens de couplage de l'objet et s'étendent suivant l'axe d'emboitement dudit élément support avec le bouchon de manière à coopérer avec la paroi du bouchon pour l'emboitement à recouvrement partiel du bouchon et de l'élément support.

En particulier, le bouchon 3 recouvre les ailettes 27 de sorte que les ailettes permettent de guider et rigidifier l'assemblage bouchon/élément support tout en protégeant le soufflet 23 par rapport au bouchon 3 pour que ledit soufflet ne soit pas déformé au cours de la fermeture ou de l'ouverture du bouchon par rapport à l'élément support.

L'espace entre les ailettes permet à l'opérateur d'accéder au soufflet pour le comprimer afin d'introduire ou libérer l'objet dans l'orifice du mandrin.

Selon un autre mode de réalisation de l'invention illustré à la figure 4, les moyens 26 pour le couplage de l'objet à l'élément support présentent une configuration d'emprisonnement de l'objet dans une orientation donnée dudit objet et une configuration de libération par pivotement dudit objet. On peut prévoir que les moyens de couplage 26 comprennent un logement dans lequel l'objet est maintenu par serrage élastique ou encliquetage. Dans cet exemple illustré à la figure 4, l'objet est une vis cervicale 7 dont la tête 70 est apte à coopérer avec l'instrument 6 pour permettre à l'opérateur de faire pivoter le corps de la vis 7 afin de la libérer des moyens de couplage 26. En particulier la libération de l'objet s'effectue par pivotement de l'objet autour d'un axe orthogonal à l'axe du logement correspondant à l'axe longitudinal de l'objet.

Selon un mode de réalisation particulier de l'invention illustré aux figures 3 et 4, l'élément support 2 et les moyens 24, 25 ; 26 pour le couplage de l'objet à l'élément support sont configurés pour maintenir l'objet suspendu à travers lesdits moyens de couplage, de manière à permettre de visualiser la longueur dudit objet à l'état couplé dudit objet à l'élément support 2.

A cet effet, l'élément support comprend une partie en forme générale de potence dont la partie haute est équipée des moyens de couplage, ce qui permet pour un objet de type vis, de suspendre ladite vis par son corps à une hauteur proche de la tête de vis, de sorte que la vis est maintenue de manière fiable sur l'élément support tout en permettant à l'opérateur de visualiser la longueur du corps de vis.

En effet, dans l'exemple illustré à la figure 3, la valve 25 est positionnée au niveau de la partie haute de la potence de sorte que l'objet qui traverse la valve peut s'étendre dans l'espace laissé libre entre la valve et la partie basse de la potence. Ainsi, la longueur de l'objet reste visible et l'objet peut être correctement maintenu.

Le maintien suspendu de l'objet permet également, dans le cas d'un objet formé de plusieurs parties articulées entre elles, de maintenir ledit objet au niveau de ladite articulation pour l'immobiliser. C'est le cas illustré à la figure 3 pour lequel l'objet suspendu à travers la valve est une vis 5' dite "tulipe" dont la tête 50' est articulée au corps par une liaison rotule. Ladite vis peut être engagée dans l'orifice de la valve jusqu'à ce que sa tête vienne en butée contre le bord de l'orifice, ce qui permet de bien caler la vis sans risque de mobilité du corps de la vis par rapport à sa tête.

Dans l'exemple illustré aux figures, lesdits éléments 2, 3, 4 de l'emballage s'emboitent à recouvrement partiel entre eux par simple friction, par exemple par pression ou serrage élastique. Un tel emboitement à recouvrement partiel par simple friction permet de procéder au déboitement desdits éléments par simple traction de l'un ou de chacun des opérateurs sur un élément de l'emballage selon un geste naturel. On pourrait également envisager un emboîtement à recouvrement partiel par clipsage.

En variante, l'emboitement à recouvrement partiel pourrait être réalisé par vissage. Dans le cas du vissage, le filet et le taraudage ménagés sur les éléments qui s'emboitent sont configurés de manière à ne permettre le vissage desdits éléments entre eux qu'une seule fois. En particulier, on peut prévoir que le filetage et/ou le taraudage se déforment lors du vissage et du dévissage de sorte qu'un nouveau vissage des deux éléments entre eux n'est plus possible.

Préférentiellement, les chambres 7 et 8 sont imperméables au moins aux bactéries. On peut prévoir qu'elles soient également imperméables à tout fluide. En variante, on peut prévoir que l'une et/ou l'autre, de préférence au moins la chambre de présentation 7 de l'objet, soit perméable uniquement aux gaz pour permettre une stérilisation gazeuse de la ou des chambres.

Ainsi on peut prévoir, selon un mode de réalisation particulier de l'invention, qu'au moins une partie de la paroi du bouchon 3 qui délimite une partie de la chambre 7 de présentation de l'objet 5 est formée en matériau perméable au gaz mais imperméable aux bactéries de manière à permettre une stérilisation gazeuse de la chambre de présentation et de l'objet, par exemple une pièce médicale, par exemple avec de l'oxyde d'éthylène ou par vapeur. Une telle conception de l'emballage permet de stériliser la ou les chambres autrement que par rayonnement.

On peut ainsi prévoir que le bouchon soit fermé à son extrémité, opposée à celle qui recouvre partiellement l'élément support, par une membrane adaptée à la stérilisation gazeuse.

Avantageusement, ledit objet 5 est couplé à l'élément support 2 de telle sorte que son extrémité de préhension 50 est libre et orientée du côté opposé à la partie de saisie 20 de l'élément support 2 afin que la zone de préhension se présente face à l'instrument de saisie de l'objet, ce qui permet une saisie aisée et rapide de l'objet.

L'élément support 2 et/ou le bouchon 3 et/ou la coiffe 4 présentent une partie, de préférence formée par une membrane, imperméable aux bactéries mais perméable aux gaz pour permettre une stérilisation gazeuse de ladite première chambre 7 et/ou de ladite deuxième chambre 8.

Le procédé de déconditionnement d'un objet contenu dans un emballage tel que décrit ci-dessus est détaillé ci-après à l'aide des figures 1A, 1B et 1C dans le cadre du passage à un opérateur "propre", ici le chirurgien au niveau du bloc opératoire, d'une pièce médicale, par l'intermédiaire de deux autres opérateurs dits "sales" c'est-à-dire travaillant dans un milieu potentiellement contaminé.

Le premier opérateur saisit d'une main MS1 dite sale, car potentiellement contaminée, l'emballage complet contenant la pièce médicale 5, par l'extrémité libre 40 de la coiffe 4. Ledit premier opérateur tend alors l'emballage au deuxième opérateur qui le saisit d'une main MS2 dite également sale par l'extrémité libre 30 du bouchon. Chacun des opérateurs tenant une extrémité de l'emballage dans la main, au moins l'un des deux tire l'emballage vers lui de sorte que la coiffe se sépare du bouchon tandis que le bouchon auquel est couplé l'élément support de la pièce médicale reste dans la main MS2 du deuxième opérateur. La distance entre les extrémités 30 et 40 de l'emballage permet au moment de l'ouverture de la chambre 8 par séparation de la coiffe et du bouchon de réduire le risque de contamination de l'élément support.

Comme rappelé ci-dessus, le premier et le deuxième opérateurs peuvent être la même personne.

La partie de saisie de l'élément support ainsi découverte reste propre et peut être présentée au troisième opérateur, à savoir le chirurgien, qui saisit d'une main dite propre MP1 ladite extrémité propre de l'élément support opposée à l'extrémité 30 du bouchon tenue par la main MS2 du deuxième opérateur.

Au moins l'un des deuxième et troisième opérateurs tire vers lui le sous-ensemble formé de l'élément support et du bouchon de sorte que la chambre 7 de présentation de l'objet s'ouvre par séparation dudit élément support 2 tenu par la main MP1 du troisième opérateur par rapport au bouchon 3 qui reste dans la main MS2 du deuxième opérateur.

Le troisième opérateur peut alors désactiver, par sa main MP1 qui tient ledit élément support, les moyens de couplage sans lâcher ledit élément support et, de son autre main propre MP2, saisir l'objet par son extrémité libre 50 à l'aide d'un instrument 6 pour l'implanter dans le corps du patient.

Dans l'exemple illustré aux figures, l'élément support délimite une cavité ouverte logeant l'objet 5 qui est destinée à être refermée par le bouchon 3.

Selon une variante de réalisation non illustrée aux figures, ledit élément support délimite à lui seul ladite première chambre renfermant ledit objet. A cet effet, ledit élément support est formé d'au moins deux pièces couplables/ désaccouplables l'une par rapport à l'autre, de préférence par emboitement à recouvrement partiel, pour former ladite première chambre renfermant ledit objet. Autrement dit, l'une des pièces dudit élément support est destinée à refermer la cavité ouverte formée par l'autre pièce.

Selon cette variante, ledit bouchon est formé par un corps creux dont la partie de fond comprend des moyens de couplage avec l'élément support.

Avantageusement, les moyens de couplage sont configurés de manière à permettre le couplage de l'objet avec la pièce de l'élément support qui forme la partie de saisie dudit élément support, c'est-à-dire la pièce de l'élément support qui n'est pas directement couplée au bouchon. Le bouchon et l'élément support s'emboitent par enfoncement à force d'une partie d'extrémité de l'élément support dans une partie creuse correspondante du bouchon. A cet effet, la partie creuse du bouchon présente des ergots qui sont destinés à être écrasés par l'élément support à l'état enfoncé dudit élément support dans le bouchon. Lesdits ergots sont répartis sur la face périphérique interne dudit bouchon autour de l'axe du bouchon.

Selon une telle variante de réalisation de l'élément support, le procédé décrit ci-dessus peut être adapté de la manière suivante. Après que l'élément support 2, tenu par une personne propre, a été séparé par rapport au bouchon 3 tenu par une personne supposée contaminée, on peut prévoir que l'élément support 2 qui renferme l'objet 5 soit mis dans une zone d'attente, en attente de l'utilisation dudit objet 5. Puis, ladite personne propre, ou une autre personne propre, peut ouvrir la première chambre 7 définie par ledit élément support 2 en séparant, par exemple par dévissage, les deux pièces qui forment ledit élément support pour saisir ladite pièce médicale.

Ainsi, la pièce médicale n'a été ni touchée ni lâchée au cours de son déconditionnement. En outre, la chambre contenant la pièce médicale n'est ouverte qu'en zone propre.

Grâce à la coiffe qui délimite une chambre de protection de la partie de saisie 20 de l'élément support 2, la personne propre touche une partie propre de l'emballage. En effet, les parties sales de l'emballage, à savoir le bouchon 3 et la coiffe 4, sont restées dans les mains de la personne sale. Ainsi, on est assuré que la main de la personne qui saisit l'élément support reste propre.

Préférentiellement, les chambres 7 et 8 sont imperméables au moins aux bactéries. On peut prévoir qu'elles soient également imperméables à tout fluide. En variante, on peut prévoir que l'une et/ou l'autre, de préférence au moins la chambre de présentation 7 de l'objet, soit perméable uniquement aux gaz pour permettre une stérilisation gazeuse de la ou des chambres.

Ainsi, on peut prévoir, selon un mode de réalisation particulier de l'invention, qu'au moins une partie de la paroi du bouchon 3 qui délimite une partie de la chambre 7 de présentation de l'objet 5 est formée en matériau perméable au gaz, mais imperméable aux bactéries de manière à permettre une stérilisation gazeuse de la chambre de présentation et de l'objet, par exemple une pièce médicale, par exemple avec de l'oxyde d'éthylène ou par vapeur. Une telle conception de l'emballage permet de stériliser la ou les chambres autrement que par rayonnement.

En particulier, on peut prévoir que le bouchon soit fermé à son extrémité, opposée à celle qui recouvre partiellement l'élément support, par une membrane adaptée à la stérilisation gazeuse.

## Revendications

1. Emballage (1) pour objet, tel qu'une pièce médicale, de préférence stérilisée, ledit emballage comprenant :
- un élément support (2) muni de moyens (21 ; 22, 23 ; 24, 25 ; 26) pour le couplage dudit objet (5) audit élément support (2), lesdits moyens pour le couplage étant désactivables pour permettre la saisie dudit objet ;
- un premier élément de protection (3) creux, appelé bouchon, couplable à l'élément support (2) dudit objet (5), de préférence par emboitement à recouvrement partiel, de telle sorte que l'élément support (2) présente une partie (20), dite partie de saisie, qui s'étend en saillie dudit bouchon (3),
ledit élément support (2) délimitant à lui seul ou en coopération avec le bouchon (3) une première chambre (7) renfermant ledit objet,
- un deuxième élément de protection (4) creux, appelé coiffe, ladite coiffe (4) étant couplable au bouchon (3), de préférence par emboitement à recouvrement partiel, de manière à délimiter en coopération avec ledit bouchon (3) une deuxième chambre (8) à l'intérieur de laquelle s'étend ladite partie de saisie (20) de l'élément support (2),
**caractérisé en ce que**, à l'état couplé de la coiffe (4) au bouchon (3), une partie (30), dite partie de saisie, du bouchon (3) s'étend en saillie de la coiffe (4).

2. Emballage (1) selon la revendication 1, **caractérisé en ce que** le bouchon (3) et la coiffe (4) sont formés chacun par un corps creux allongé ouvert à une extrémité et fermé à l'autre extrémité.

3. Emballage (1) selon l'une des revendications précédentes, **caractérisé en ce que** les moyens pour le couplage de l'objet (5) à l'élément support (2) comprennent un corps déformable (21), appelé valve, présentant un orifice, de préférence de forme ovale, apte à passer, par déformation dudit corps, d'une configuration dite de rétention dans laquelle ledit orifice de la valve (21) présente une forme et/ou des dimensions permettant d'enserrer l'objet (5), à une configuration dite de libération dans laquelle ledit orifice présente une forme et/ou des dimensions permettant de libérer ledit objet (5) par rapport audit orifice de la valve (21).

4. Emballage (1) selon l'une des revendications précédentes, **caractérisé en ce que** les moyens (26) pour le couplage de l'objet à l'élément support présentent une configuration d'emprisonnement de l'objet dans une orientation donnée dudit objet et une configuration de libération par pivotement dudit objet.

5. Emballage (1) selon l'une des revendications précédentes, du type pour lequel ledit objet présente un corps allongé,
**caractérisé en ce que** lesdits moyens pour le couplage de l'objet à l'élément support comprennent un corps allongé déformable (22), appelé mandrin, le long et à l'intérieur duquel est ménagé un orifice d'insertion de l'objet,
lesdits moyens comprenant également un soufflet (23), entourant le mandrin (22), apte à passer d'une position déployée des plis de soufflet dans laquelle ledit soufflet appuie sur le mandrin (22) de manière à resserrer l'orifice d'insertion autour de l'objet (5), à une position comprimée desdits plis de soufflet dans laquelle le mandrin (22) est libre de contrainte par rapport au soufflet de manière à permettre le déplacement de l'objet par rapport audit orifice d'insertion du mandrin.

6. Emballage (1) selon la revendication 5, **caractérisé en ce que** ledit élément support (2) comprend au moins deux ailettes (27, 28) écartées l'une de l'autre et qui s'étendent de part et d'autre et le long de l'ensemble formé du mandrin (22) et du soufflet (23).

7. Emballage (1) selon l'une des revendications précédentes, **caractérisé en ce que** l'élément support (2) et les moyens pour le couplage de l'objet à l'élément support sont configurés pour maintenir l'objet (5) suspendu à travers lesdits moyens de couplage (24, 25), de manière à permettre de visualiser la longueur dudit objet à l'état couplé dudit objet (5) à l'élément support (2).

8. Emballage (1) selon l'une des revendications précédentes, **caractérisé en ce que** lesdits éléments de support (2) et de protection (3, 4) de l'emballage s'emboitent à recouvrement partiel entre eux par friction et/ou par vissage.

9. Emballage (1) selon l'une des revendications 1 à 8, **caractérisé en ce que**, ledit élément support (2) délimitant à lui seul ladite première chambre (7) renfermant ledit objet (5), ledit élément support (2) est formé d'au moins deux pièces couplables/désaccouplables l'une par rapport à l'autre, de préférence par emboîtement à recouvrement partiel, pour former ladite première chambre renfermant ledit objet (5).

10. Emballage (1) selon l'une des revendications 1 à 8, **caractérisé en ce que**, ledit élément support (2) délimitant en coopération avec le bouchon (3) ladite première chambre (7) renfermant ledit objet (5), ledit élément support (2) délimite une cavité ouverte destinée à être refermée par ledit bouchon (3).

11. Emballage (1) selon l'une des revendications précédentes, **caractérisé en ce que** ledit bouchon (3), et éventuellement la coiffe (4), présente(nt) au moins un emplacement (32) prédéfini de positionnement d'au moins un doigt d'une personne.

12. Emballage (1) selon l'une des revendications précédentes, **caractérisé en ce que** l'élément support (2) et/ou le bouchon (3) et/ou la coiffe (4) présente(nt) une partie, de préférence formée par une membrane, imperméable aux bactéries mais perméable aux gaz pour permettre une stérilisation gazeuse de ladite première chambre (7) et/ou de ladite deuxième chambre (8).

13. Procédé de déballage d'un objet, de préférence stérilisé, contenu dans un emballage (1) conforme à l'une des revendications précédentes, **caractérisé en ce que** ledit procédé comprend les étapes suivantes :
a)- séparation de la coiffe (4) par rapport au bouchon (3) par une première personne de manière à découvrir l'élément support (2),
b)- saisie de l'élément support (2) par une deuxième personne distincte de ladite première personne,
c)- séparation dudit élément support (2) par rapport au bouchon (3) tenu par la première personne.

14. Procédé de déballage selon la revendication 13, ledit emballage étant conforme à la revendication 9, **caractérisé en ce que** ledit procédé comprend les étapes supplémentaires suivantes :
d)- éventuellement, mise en attente dudit élément support (2) qui renferme l'objet (5) dans une zone d'attente, en attente de l'utilisation dudit objet (5),
e)- ouverture de la première chambre (7) définie par ledit élément support (2) pour en extraire ledit objet (5).

15. Ensemble (1) comprenant un objet (5), tel qu'une pièce médicale de préférence stérilisée, et un emballage selon l'une des revendications 1-12, ledit emballage contenant ledit objet (5) couplé audit élément support (2), ledit objet (5) présentant une extrémité (50) servant à sa préhension par exemple par un instrument (6),
**caractérisé en ce que** ledit objet (5) est couplé à l'élément support (2) de telle sorte que son extrémité de préhension (50) est libre et orientée du côté opposé à la partie de saisie (20) de l'élément support (2).

## Patentansprüche

1. Verpackung (1) für ein Objekt wie ein vorzugsweise sterilisiertes medizinisches Teil, wobei die Verpackung umfasst:
- ein mit Kopplungsmitteln (21; 22, 23; 24, 25; 26) des Objekts (5) an das Stützelement (2) ausgestattetes Stützelement (2), wobei die Kopplungsmittel für das Ergreifen des Objekts deaktivierbar sind,
- ein erstes hohles, als Stopfen bezeichnetes Schutzelement (3), das am Stützelement (2) des Objekts (5) vorzugsweise durch Rasten mit teilweiser Abdeckung koppelbar ist, so dass das Stützelement (2) einen als Erfassungsabschnitt bezeichneten Abschnitt (20) aufweist, der aus dem Stopfen (3) herausragt,
wobei das Stützelement (2) allein oder in Zusammenarbeit mit dem Stopfen (3) eine erste Kammer (7) begrenzt, die das Objekt abschließt,
- ein zweites, als Haube bezeichnetes Schutzelement (4),
wobei die Haube (4) am Stopfen (3) vorzugsweise durch Rasten mit teilweiser Abdeckung koppelbar ist, so dass in Zusammenarbeit mit dem Stopfen (3) eine zweite Kammer (8) begrenzt wird, im Innern derselben sich der Erfassungsabschnitt (20) des Stützelements (2) erstreckt,
**dadurch gekennzeichnet, dass** im gekoppelten Zustand der Haube (4) am Stopfen (3) ein als Erfassungsabschnitt bezeichneter Abschnitt (30) des Stopfen (3) aus der Haube (4) herausragt.

2. Verpackung (1) nach Anspruch 1, **dadurch gekennzeichnet, dass** der Stopfen (3) und die Haube (4) jeweils von einem länglichen, an einem Ende offenen und am anderen Ende geschlossenen Körper gebildet sind.

3. Verpackung (1) nach einem der vorangehenden Ansprüche, **dadurch gekennzeichnet, dass** die Mittel für das Koppeln des Objekts (5) am Stützelement (2) einen als Ventil bezeichneten verformbaren Körper (21) umfassen, der eine vorzugsweise ovale Öffnung aufweist, der imstande ist, durch Verformung des Körpers aus einer Haltekonfiguration, in welcher die Öffnung des Ventils (21) eine Form und/oder Abmessungen aufweist, die erlauben, das Objekt (5) zu umspannen, in eine Freigabekonfiguration, in welcher die Öffnung eine Form und/oder Abmessungen aufweist, die erlauben, das Objekt (5) im Verhältnis zur Öffnung des Ventils (21) freizugeben, zu wechseln.

4. Verpackung (1) nach einem der vorangehenden Ansprüche, **dadurch gekennzeichnet, dass** die Mittel (26) für das Koppeln des Objekts am Stützelement eine Umfassungskonfiguration des Objekts in einer bestimmten Ausrichtung des Objekts und eine Freigabekonfiguration durch Drehen des Objekts aufweisen.

5. Verpackung (1) nach einem der vorangehenden Ansprüche des Typs, für den das Objekt einen länglichen Körper aufweist,
**dadurch gekennzeichnet, dass** die Mittel für das Koppeln des Objekts am Stützelement einen als Aufnahme bezeichneten verformbaren länglichen Körper (22) umfassen, entlang und im Innern dessen eine Einsetzöffnung des Objekt ausgebildet ist,
wobei die Mittel ebenfalls einen die Aufnahme (22) umgebenden Balg (23) umfassen, der imstande ist, von einer Position, in der die Falten des Balgs entfaltet sind, in welcher sich der Balg auf der Aufnahme (22) abstützt, so dass die Einsetzöffnung um das Objekt (5) gespannt ist, in eine Position, in der die Falten des Balgs komprimiert sind, in welcher die Aufnahme (22) im Verhältnis zum Balg spannungsfrei ist, um die Verlagerung des Objekts im Verhältnis zur Einsetzöffnung der Aufnahme zu erlauben, zu wechseln.

6. Verpackung (1) nach Anspruch 5, **dadurch gekennzeichnet, dass** das Stützelement (2) mindestens zwei Flügel (27, 28) umfasst, die voneinander beabstandet sind und die sich auf der einen und der anderen Seite und entlang der Einheit erstrecken, die von der Aufnahme (22) und dem Balg (23) gebildet ist.

7. Verpackung (1) nach einem der vorangehenden Ansprüche, **dadurch gekennzeichnet, dass** das Stützelement (2) und die Mittel für das Koppeln des Objekt am Stützelement konfiguriert sind, um das Objekt (5) hängend durch die Kopplungsmittel (24, 25) zu halten, so dass die Länge des Objekts im gekoppelten Zustand des Objekts (5) am Stützelement (2) visualisierbar ist.

8. Verpackung (1) nach einem der vorangehenden Ansprüche, **dadurch gekennzeichnet, dass** die Stütz- (2) und Schutzelemente (3, 4) der Verpackung mit teilweiser Abdeckung untereinander durch Reiben und/oder durch Schrauben ineinander rasten.

9. Verpackung (1) nach einem der Ansprüche 1 bis 8, **dadurch gekennzeichnet, dass**, wobei das Stützelement (2) allein die erste Kammer (7), die das Objekt (5) einschließt, begrenzt, das Stützelement (2) von mindestens zwei im Verhältnis zueinander koppelbaren/entkoppelbaren Teilen, vorzugsweise durch Rasten mit teilweiser Abdeckung, gebildet ist, um die erste Kammer zu bilden, die das Objekt (5) einschließt.

10. Verpackung (1) nach einem der Ansprüche 1 bis 8, **dadurch gekennzeichnet, dass**, wobei das Stützelement (2) in Zusammenarbeit mit dem Stopfen (3) die erste Kammer (7), die das Objekt (5) einschließt, begrenzt, das Stützelement (2) einen offenen Hohlraum begrenzt, der bestimmt ist, von dem Stopfen (3) verschlossen zu sein.

11. Verpackung (1) nach einem der vorangehenden Ansprüche, **dadurch gekennzeichnet, dass** der Stopfen (3) und eventuell die Haube (4) mindestens eine vorbestimmte Positionierungsstelle (32) mindestens eines Fingers einer Person aufweist/aufweisen.

12. Verpackung (1) nach einem der vorangehenden Ansprüche, **dadurch gekennzeichnet, dass** das Stützelement (2) und/oder der Stopfen (3) und/oder die Haube (4) einen vorzugsweise von einer für Bakterien undurchdringlichen, aber für Gase durchdringlichen Membran gebildeten Abschnitt aufweist/aufweisen, um eine Gassterilisation der ersten Kammer (7) und/oder der zweiten Kammer (8) zu erlauben.

13. Verfahren zum Entpacken eines vorzugweise sterilisierten Objekts, das in einer Verpackung (1) nach einem der vorangehenden Ansprüche enthalten ist, **dadurch gekennzeichnet, dass** das Verfahren die folgenden Schritte umfasst:
a) Trennen der Haube (4) im Verhältnis zum Stopfen (3) durch eine erste Person, um das Stützelement (2) freizulegen,
b) Erfassen des Stützelements (2) durch eine zweite Person, die sich von der ersten Person unterscheidet,
c) Trennen des Stützelements (2) im Verhältnis zum von der ersten Person gehaltenen Stopfen (3).

14. Verfahren zum Entpacken nach Anspruch 13, wobei die Verpackung Anspruch 9 entspricht, **dadurch gekennzeichnet, dass** das Verfahren die folgenden zusätzlichen Schritte umfasst:
d) eventuelles Versetzen in Wartestellung des Stützelements (2), welches das Objekt (5) einschließt, in einer Wartezone in Erwartung der Verwendung des Objekts (5),
e) Öffnen der ersten von dem Stützelement (2) definierten Kammer (7), um daraus das Objekt (5) zu entnehmen.

15. Einheit (1), umfassend ein Objekt (5) wie ein vorzugsweise sterilisiertes medizinisches Teil und eine Verpackung nach einem der Ansprüche 1 bis 12, wobei die Verpackung das am Stützelement (2) gekoppelte Objekt (5) enthält, wobei das Objekt (5) ein Ende (50) aufweist, das zu seinem Ergreifen beispielsweise durch ein Instrument (6) dient,
**dadurch gekennzeichnet, dass** das Objekt (5) derart am Stützelement (2) gekoppelt ist, dass sein Griffende (50) frei ist und zu der Seite zeigt, die dem Erfassungsabschnitt (20) des Stützelements (2) gegenüberliegt.

## Claims

1. A package (1) for an object, such as a medical part, preferably sterilized, said package comprising:
- a supporting element (2) provided with means (21; 22, 23; 24, 25; 26) for coupling said object (5) with said supporting element (2), said means for the coupling capable of being deactivated for allowing the grasping of said object;
- a first hollow protective element (3) called a plug which may be coupled with the supporting element (2) of said object (5), preferably by jointing with partial overlap, so that the supporting element (2) has a portion (20), called grasping portion, which extends, protruding from said plug (3),
said supporting element (2) delimiting by itself or by cooperating with the plug (3) a first chamber (7) containing said object,
- a second hollow protective element (4), called a cap,
said cap (4) being able to be coupled to the plug (3) preferably by jointing with partial overlap, so as to delimit by cooperating with said plug (3) a second chamber (8) inside which extends said grasping portion (20) of the supporting element (2),
**characterized in that**, in the coupled state of the cap (4) with the plug (3), a portion (30), called grasping portion, of the plug (3) extends protruding from the cap (4).

2. The package (1) according to claim 1, **characterized in that** the plug (3) and the cap (4) are each formed of by an elongated hollow body open at one end and closed at the other end.

3. The package (1) according to one of the preceding claims,
**characterized in that** the means for coupling the object (5) with the supporting element (2) comprise a deformable body (21), called a valve, having an orifice, preferably of an oval shape, capable of passing, by deformation of said body, from a so-called retention configuration wherein said orifice of the valve (21) has a shape and/or dimensions giving the possibility of clasping the object (5), to a so-called release configuration wherein said orifice has a shape and/or dimensions giving the possibility of releasing said object (5) relatively to said orifice of the valve (21).

4. The package (1) according to one of the preceding claims, **characterized in that** the means (26) for coupling the object with the supporting element have a configuration for confining the object in a given orientation of said object and a configuration for releasing said object by pivoting.

5. The package (1) according to one of the preceding claims, of the time for which said object has an elongated body,
**characterized in that** said means for coupling the object with the supporting element comprise a deformable elongated body (22), called a mandrel, along an inside which an orifice is made for inserting the object,
said the means also comprising bellows (23), surrounding the mandrel (22) cult, able to pass from a deployed position of the bellows' folds in which said bellows bear upon the mandrel (22) so as to tighten the insertion orifice around the object (5), in a compressed position of said bellows' folds wherein the mandrel (22) is stress-free relatively to the bellows so as to allow displacement of the object relatively to said orifice for inserting the mandrel.

6. The package (1) according to claim 5, **characterized in that** said supporting element (2) comprises at least two fins (27, 28) separated from each other and which extends the on either side and along the assembly formed with the mandrel (22) and the bellows (23).

7. The package (1) according to one of the preceding claims,
**characterized in that** the supporting element (2) and the means for coupling the object with the supporting element are configured for maintaining the object (5) suspended through said coupling means (24, 25), so as to give the possibility of viewing the length of said object (5) in the coupled state of said object (5) with the supporting element (2).

8. The package (1) according to one of the preceding claims,
**characterized in that** said supporting (2) and protective (3,4) elements of the package fit together with partial overlapping of each other by friction and/or by screwing.

9. The package (1) according to claims 1 to 8, **characterized in that** said supporting element (2) delimiting by itself said first chamber (7) containing said object (5), said supporting element (2) is formed with at least two parts which may be coupled/uncoupled with each other, preferably coupled with partial overlapping, in order to form said first chamber containing said object (5).

10. The package (1) according to claims 1 to 8, **characterized in that** said supporting element (2) delimiting by cooperating with the plug (3), said first chamber (7) containing said object (5), said supporting element (2) delimits an open cavity in tended to be closed by said plug (3).

11. The package (1) according to one of the preceding claims, **characterized in that** said plug (3), and optionally the cap (4) have at least one predefined location (32) for positioning at least one finger of a person.

12. The package (1) according to one of the preceding claims, **characterized in that** the supporting element (2) and/or the plug (3) and/or the cap (4) have a portion, preferably formed by a membrane, in an impervious to bacteria but impervious to gases for allowing gas sterilization of said first chamber (7) and/or of said second chamber (8).

13. A method for unpacking an object, preferably sterilized, contained in a package (1) according to one of the preceding claims, **characterized in that** said method comprises the following steps:
a) separation of the cap (4) relatively to the plug (3) by a first person so as to uncover the supporting element (2),
b) grasping the supporting element (2) by a second person distinct from said first person,
c) separating said supporting element (2) relatively to the plug (3) held by the first person.

14. The unpacking method according to claim 13, said package being according to claim 9, **characterized in that** said method comprises the following additional steps:
d) optionally, putting on hold said supporting element (2) which contains the object (5) in a waiting area, awaiting the use of said object (5),
e) opening the first chamber (7) defined by a said supporting element (2) in order to extract said object (5) therefrom.

15. An assembly (1) comprising an object (5), such as a medical part preferably sterilized, and a package according to one of claims 1 to 12, said package containing said object (5) coupled with said supporting element (2), said object (5) having an end (50) used for its grasping for example by an instrument (6), **characterized in that** said object (5) is coupled with the supporting element (2) so that its grasping end (50) is free and oriented on the side opposite to the grasping portion (20) of the supporting element (2).
